# EUROPÄISCHE PATENTSCHRIFT

(11) **EP 0 564 953 B1**
(45) Veröffentlichungstag und Bekanntmachung des Hinweises auf die Patenterteilung: **28.12.1994**
(21) Anmeldenummer: 93105198.1
(22) Anmeldetag: 29.03.1993
(51) Int. Cl.: A61M 13/00

(54) **Erwärmungsvorrichtung für Insufflationsgerät**
Heating device for insufflation apparatus
Dispositif de chauffage pour appareil d'insufflation

(30) Priorität: 09.04.1992 DE 4211986
(43) Veröffentlichungstag der Anmeldung: 13.10.1993
(73) Patentinhaber: WISAP GESELLSCHAFT FÜR WISSENSCHAFTLICHEN APPARATEBAU MBH, D-82054 Sauerlach (DE)
(72) Erfinder: Semm, Kurt, o.Prof. Dr.med. Dr.med.vet.h.c., W-2300 Kiel (DE)
(74) Vertreter: Heim, Hans-Karl, Dipl.-Ing.

(56) Entgegenhaltungen:
- EP-A- 0 201 985
- DE-A- 2 454 349
- US-A- 3 982 533
- US-A- 5 006 109

## Beschreibung

Die Erfindung betrifft eine Erwärmungsvorrichtung gemäß Oberbegriff des Anspruchs 1.

Bei medizinischen Untersuchungen oder chirurgischen Eingriffen im Bereich des Abdomen oder Thorax, insbesondere bei der Laparoskopie und Pelviskopie, werden heute Insufflationsgeräte eingesetzt, mit deren Hilfe ein Gas, z.B. CO₂ Gas oder N₂O-Gas, in die entsprechende Körperhöhle insuffliert wird. Dieses mit einem relativ geringen Überdruck von z.B. 14 mm/Hg bis 50 mm/Hg in die entsprechende Körperhöhle zugeführte Gas, das vom Körper selbst postoperativ resorbiert wird, gestattet es dem Chirurgen, den entsprechenden Organbereich bzw. Gewebebereich endoskopisch untersuchen oder chirurgisch behandeln zu können.

Diese Insufflationsgeräte sind daher in der jüngeren endoskopischen Chirurgie hinreichend bekannt.

Ein Problem, das bisher noch nicht gelöst worden ist, besteht darin, daß das über verschiedene Druckreduzierungsstufen auf den erforderlichen und gewünschten Druck reduzierte Gas derzeit am Ausströmnippel des Insufflationsgerätes etwa eine Temperatur von 16 bis 24°C aufweist und mit dieser Temperatur, die medizinisch gesehen weit unter der Körpertemperatur liegt, in den Körper insuffliert wird.

Diese relativ niedrige Temperatur resultiert daraus, daß das Gas aus einer Flüssigkeitsdruckflasche bei einem Druck von etwa 60 bar und Raumtemperatur von etwa 23°C dem Insufflationsschlauch zugeführt wird. Das aus Flüssigkeit durch Druckreduzierung über mehrere Stufen entspannte Gas führt aufgrund der volumenmäßigen Expansion des Gases zunächst zu einer extremen Temperaturabsenkung bis weit unter 0°C.

Zwar kann im weiteren Leitungsverlauf im Insufflationsgerät vornehmlich aber im Insufflationsschlauch die Temperatur auf Raumtemperatur von etwa 21°C angehoben werden, so daß das in den Körper insufflierte CO₂-Gas etwa diese Temperatur aufweist. Es liegt damit aber noch erheblich unter der Körpertemperatur von 37°C.

Der Temperaturunterschied zwischen dem insufflierten Gas und dem Gewebe mit Körpertemperatur bei 37°C kann zu katarrhalischen Effekten im Abdomen und damit zu Schmerzen unspezifischer Art führen, die medizinisch noch nicht genau diagnostizierbar sind.

Es besteht daher ohne Zweifel medizinisch das Bedürfnis, diesen Temperaturunterschied zwischen dem zu insufflierenden Gas und der Körpertemperatur zu egalisieren, um medizinisch jegliche unspezifische Disposition für den Patienten zu vermeiden.

Versuche, die Erwärmung des CO₂-Gases während einer Insufflation mittels eines Warmwasserbades zu erreichen, indem das Gas durch eine V2A-Leitung bei einer Temperatur von etwa 50°C geführt wird, haben einen schlechten Wirkungsgrad bei der Erwärmung gezeigt, wobei auch die Länge der Leitung unbefriedigend war.

Eine weitere Alternative, das Gas durch eine kästchenartige Erwärmungsbox zu führen, in der ein Heizstern vorgesehen ist, mußte aufgrund verschiedener problematischer Effekte aufgegeben werden. Diesbezügliche Probleme liegen vor allem darin, daß das Gas zu stark aufgeheizt werden kann, was gerade bei einer sehr niedrigen Gasströmung (Flow) und einem nachfolgend erforderlichen hohen Flow auftreten kann.

Zudem jedoch ist die Erwärmungsbox unter den Gesichtspunkten medizinisch steriler Geräte unbrauchbar, da die in der Erwärmungsbox entstehende Feuchtigkeit und Wärme ein ideales Medium für Pilze wäre, so daß eine erhebliche Kontaminationsgefahr darin gesehen werden muß.

Hinzu treten selbstverständlich die medizinisch erforderlichen Sterilisationskriterien, wobei die Geräte z.B. bei einer Dampfsterilisation einer Temperatur von ca. 134°C ausgesetzt werden. Denkbar ist jedoch auch eine Gassterilisation z.B. mit Ethylenoxid bei etwa 60°C, was jedoch über eine lange Zeitdauer erfolgen muß und in Zukunft auch nicht mehr zugelassen sein wird.

Eine gattungsgemäße Vorrichtung ist aus der US 5,006,109 bekannt. Bei dieser bekannten Vorrichtung ist eine Heizeinrichtung zur Erwärmung des Gases in einem Gerätegehäuse angeordnet. Aufgrund der Abkühlung des Gases nach Durchgang durch die Heizeinrichtung kann die Gastemepratur nicht mit einer wünschenswerten Genauigkeit eingestellt werden.

In der EP-A-0 201 985 ist ein Inhalationsgerät offenbart, bei dem zur Vermeidung eines Wärmeverlustes um eine Schlauchleitung eine Heizspirale gewickelt ist. Eine derartige Heizeinrichtung, insbesondere zur Blutleitung, ist auch aus der DE-A-24 54 349 bekannt.

Der Erfindung liegt die **Aufgabe** zugrunde, eine einfache Erwärmungsvorrichtung für ein Insufflationsgerät zu schaffen, mit der die Temperatur des zu insufflierenden Gases besonders genau eingestellt werden kann und welche gegebenenfalls im Sinne eines Nachrüstsatzes an vorhandene Insufflationsgeräte angeschlossen werden kann.

Diese Aufgabe, wird erfindungsgemäß durch eine Erwärmungsvorrichtung mit den Merkmalen des Anspruchs 1 gelöst.

Ein essentieller Gedanke der Erfindung beruht daher darauf, das zu insufflierende Gas nicht mit etwa Raumtemperatur in den biologischen Körper zu leiten, sondern das Gas weitgehend auf die Körpertemperatur anzugleichen, so daß nachteilige Auswirkungen auf den Körper vermieden werden können. Üblicherweise bedeutet dies eine Erwärmung des Gases von Raumtemperatur auf eine Temperatur im Bereich von 37°C. Die Erwärmung des Gases wird unmittelbar vor einem in den biologischen Körper führenden Insufflationsinstrument durchgeführt. Dabei wird die Erwärmung über eine längere Insufflationsstrecke, insbesondere etwa im Bereich von 1,5 bis 2,5 m durchgeführt.

Insbesondere im Hinblick auf die weitgehend zur Norm gewordenen Insufflationsgeräte, die einen einzigen Geräteausgang als Leitungsanschlußnippel für die Leitung zum Patienten wie auch gleichzeitig als Meßausgang und -eingang aufweisen, geht die Erfindung den Weg, zwischen diesem Geräteausgang und der distalseitig am Ende der Leitung vorgesehenen Veress-Nadel einen Heizschlauch vorzusehen. Dieser Heizschlauch besteht bevorzugterweise aus mehreren Schlauchmänteln, die selbst aus sterilisierbarem Material z.B. extrudiert sind. Hierfür eignet sich besonders ein Silikonmaterial oder PU-Material. Nahe am Bereich der Innenwandung des Heizschlauches, was gegebenenfalls auch zwischen den beiden Schlauchmänteln sein kann, wird ein Heizdraht, insbesondere als Heizdrahtwicklung vorgesehen, die auch direkt mitextrudiert werden kann.

Alternativ hierzu kann auch eine reine Längsorientierung von Heizdrähten vorgesehen werden, die proximal- oder distalseitig - abgesehen von einem Leiter als Rückleiter - miteinander elektrisch verbunden, z.B. verschweißt, sind.

Obwohl Silikon kein guter Wärmeleiter ist, hat es sich bei CO₂-Gas überraschenderweise gezeigt, daß die gewünschte Temperaturerhöhung über eine Heizschlauchlänge von etwa 2 m erreicht wird. Dies gilt sowohl für den Fall eines Flows von Null als auch für den Fall eines Flows im Bereich von 10 l/min bis 18 oder 20 l/min.

Dieses Phänomen einer ausreichenden Wärmeübertragung von der Heizdrahtwicklung durch die geringe Schichtdicke des Silikons auf das CO₂ -Gas wird darauf zurückgeführt, daß es sich beim CO₂ aufgrund seiner hohen spezifischen Dichte im Bereich von 1,977 kg/m³ um eine weitgehend laminare Strömung handelt. Die Wärmeaufnahme des CO₂-Gases geschieht daher primär durch Verwirbelungen an der Innenwandungsfläche des Heizschlauches.

Aufgrund dieses überraschenden Effekts folgt daher beim Flow Null auch bei längerem Stillstand nur eine relativ geringe, aber gewünschte Erwärmung. Andererseits ist bei hohen Flowraten die Verwirbelung an der Innenwandfläche sehr groß, wodurch auch die Wärmeaufnahme des CO₂-Gases im erwünschten hohen Maß gegeben ist.

Der Heizschlauch wird daher erfindungsgemäß insbesondere in einer Länge von 2 m bis 2,4 m direkt an dem Ausgangsnippel des Insufflationsgerätes angeschlossen. Distalseitig ist der Heizschlauch vorzugsweise über 1 bis 2 cm ohne Heizwicklung ausgestattet. Hierdurch ist sichergestellt, daß die angeschlossenen Elemente, wie Torsionskupplung, Veress-Nadel etc., keine ungewünschte Erwärmung erfahren und auf alle Fälle ein elektrischer Kontakt zur Heizwicklung eliminiert ist.
In diesem Bereich ist vorteilhafterweise auch ein Temperaturfühler integriert, der jedoch keine zusätzlichen Verwirbelungen bewirken sollte, da dies zu einer Meßverfälschung führen kann.

In optimaler Weise ist die Temperaturfühlerleitung mit in den Heizschlauch integriert bzw. extrudiert.

Unter Stabilitäts- und fertigungstechnischen Gesichtspunkten können die Heizdrahtwicklungen auf einer schlauchförmigen textil- oder glasfaserähnlichen Matte vorgesehen sein, so daß die Einbettung zwischen zwei Schlauchmänteln leichter möglich ist. Während der Innenmantel aus einem etwas weicheren Silikonmaterial bestehen kann, wird für den äusseren Schlauchmantel ein stabileres Silikonmaterial gewählt. Es wird jedoch sichergestellt, daß keinerlei Partikelablösung oder eine Kondensation des Gases am Innenwandungsbereich vor sich geht.

Bei einem Nachrüstsatz der Erwärmungsvorrichtung werden die Heizleiter proximalseitig an einen Trafo mit Spannungskonstanter, hinter der Gleichrichtung, angeschlossen. Sekundärseitig genügt etwa eine Leistung von 20 Watt bei einer Gleichspannung von konstant 20 V. Aufgrund der eingesetzten Gleichspannung werden daher elektromagnetische Störstrahlungen auf andere Geräte vermieden.

Im Idealfall jedoch wird die Spannungsversorgung der Heizdrahtwicklung direkt am Ausgangsnippel des Insufflationsgerätes durch kontaktiertes Aufschieben des Schlauches auf den Nippel herbeigeführt. Die Spannungsversorgung selbst ist ebenso wie die Temperaturanzeige distalseitig am Heizschlauch, in das Insufflationsgerät integriert.

Vorteilhafterweise ist der Heizdraht als Kaltleiter bzw. Widerstandsdraht ausgelegt, so daß in der Anfangsphase, insbesondere der Aufheizzeit von etwa 10 min, ein anfänglich hoher Strom gezogen wird. Mit zunehmender Erwärmung erhöht sich der Heizleiterwiderstand und der Strom sinkt auf einen etwa konstanten Wert. Hierdurch ist im Normalbetrieb auch eine kleinere Wärmeleistung erforderlich.

Elektrisch vorteilhaft ist eine dreiadrige Kabelverbindung zwischen Heizschlauch und der Spannungsversorgung; die dritte Ader dient als Masse- oder Entladungsleitung bei statischer Aufladung des Heizschlauches. Des weiteren wird dadurch eine höhere mechanische Druckbelastung an der Spannzange des Steckers erreicht.

Für eine kraftmäßige Entkopplung zwischen Veress-Nadel und Heizschlauch kann eine Torsionskupplung vorgesehen sein, die rotative Bewegungen und Kräfte zwischen Veress-Nadel und Heizschlauch ausgleicht.

Die Erfindung wird nachstehend anhand einer schematischen Darstellung näher erläutert. Es zeigen:
- Fig. 1: eine Prinzipskizze der Druckreduzierung in einem Insufflationsgerät mit ausgangsseitig vorgesehenem Heizschlauch;
- Fig. 2: eine vereinfachte Darstellung eines Insufflationsgerätes mit angeschlossenem Heizschlauch einschließlich distalseitiger Veress-Nadel und
- Fig. 3: einen Radialschnitt durch ein Ausführungsbeispiel eines Heizschlauchs.

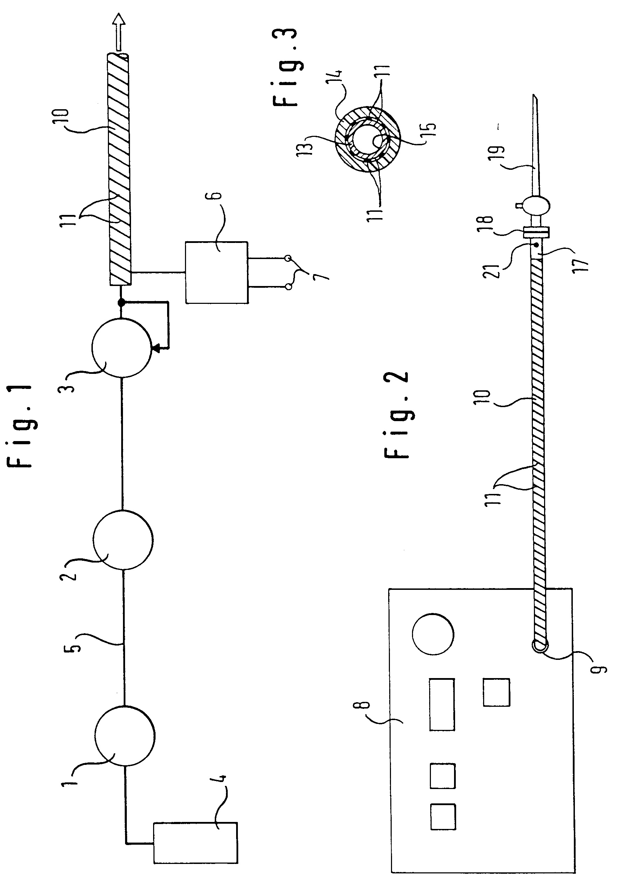

Das in Fig. 1 dargestellte Prinzip der Druckreduzierung zeigt zunächst eine Druckflasche 4 mit flüssigem CO₂-Gas. Die Temperatur des Gases entspricht etwa der Raumtemperatur bei ca. 21°C, wobei jedoch ein Flüssigkeitsdampf-Druck von raumtemperaturabhängig 40 bis 60 bar vorliegt. Dieses unter 60 bar stehende CO₂-Gas wird in einem ersten Druckminderer 1 auf etwa 0,8 bar entspannt, wobei ein Temperaturabfall bis in den Bereich von 0°C auftreten kann. Dem ersten Druckminderer nachgeschaltet ist in der Gasleitung 5 ein zweiter Druckminderer 2, der eine weitere Druckreduzierung bis auf ca. 50 mm/Kg durchführt. Dem zweiten Druckminderer 2 schließt sich dann ein Druckregler 3 an, mit dem das zu insufflierende CO₂-Gas in einem Druckbereich von 0 bis 14 mm/Hg bzw. bis 50 mm/Hg reguliert werden kann.

Am Ausgang dieses Druckreglers 3 hat das entspannte CO₂-Gas noch eine Temperatur im positiven °C-Bereich, jedoch unter der Raumtemperatur angenommen.

Damit dieses relativ kalte CO₂-Gas nicht in den Körper insuffliert wird, wird erfindungsgemäß eine Schlauchleitung 10 mit mehreren Heizdrahtwicklungen 11 am Ausgang des Insufflationsgeräts angeschlossen. Distalseitig am Heizschlauch erfolgt z.B. der Anschluß der Veress-Nadel 19.

Um den erfindungsgemäßen Heizschlauch ebenfalls bei herkömmlichen Insufflationsgeräten einsetzen zu können, kann eine separate Trafoeinheit 6 vorgesehen sein, die primärseitig an das Netz 7 mit 220 Volt und sekundärseitig über einen Spannungskonstanter 20 V Gleichspannung mit einer Ausgangsleistung von etwa 20 W liefert.

In der Darstellung nach Fig. 2 ist an einem Insufflations gerät 8, und zwar an dessen Gasanschluß 9, direkt die Schlauchleitung 10 aufgebracht. Distalseitig ist bei diesem Heizschlauch etwa ein Längenbereich 17 von 1 bis 2 cm ohne Heizdrahtwicklungen 11 ausgestattet. Der Gasanschluß 9 kann auch ein kombinierter Gas- und Meßanschluß sein.

Um Torsionskräfte bei einer Schlauchlänge von 2 m zu egalisieren, ist distalseitig eine Torsionskupplung 18 vorgesehen, die wiederum mit einer Veress-Nadel zur Insufflation des Gases in den Körper in Verbindung steht. Mit 21 ist ein Temperaturfühler, z.B. ein PT 100, angedeutet, der über eine integrierte, in der Schlauchleitung 10 vorgesehene Leitung mit dem Insufflationsgerät 8 in Verbindung steht.

Hierdurch besteht auch die Möglichkeit, in Abhängigkeit vom Flow und der am Temperaturfühler 21 ermittelten Temperatur die Heizleistung im Heizschlauch zu regeln.

Der Heizschlauch gewährleistet daher nach einer Anwärmzeit von 15 bis 20 min und einer elektrischen Beschaltung von etwa 20 Volt sekundärseitig bei etwa 1 Ampere und einer Leistung von ca. 20 Watt eine Erwärmung des CO₂-Gases nahezu unabhängig vom Flow auf einen Temperaturbereich, der etwa der Körpertemperatur entspricht.

Im Radialschnitt ist ein Ausführungsbeispiel des Heizschlauchs in Fig. 3 dargestellt. Der Heizschlauch weist hierbei einen Innenmantel 13 und einen Außenmantel 14 z.B. aus unterschiedlichen Silikonmaterialien auf. Zwischen dem Innen- und dem Außenmantel sind z.B. auf einer Textilfasermatte sechs bis acht Heizdrähte 11 über den Umfang verteilt vorgesehen. Diese Heizdrähte 11 stehen daher nicht direkt, sondern indirekt über die relativ dünne Mantelschicht mit dem durch den Heizschlauch fließenden oder stehenden CO₂-Gas in Wärmeaustausch.

## Patentansprüche

1. Erwärmungsvorrichtung für ein, in einen biologischen Körper einzuleitendes Gas, insbesondere CO₂-Gas, für ein Insufflationsgerät (8) mit einer Heizeinrichtung (11), mit der das Gas auf eine vorgesehene Temperatur erwärmbar ist, und mit einer Schlauchleitung (10), die vom Insufflationsgerät (8) zu dem biologischen Körper führt,
dadurch **gekennzeichnet,**
daß die Heizeinrichtung (11) in der Schlauchleitung (10) ausgebildet ist und
daß die Heizeinrichtung (11) steuerbar ist, so daß das Gas unmittelbar vor einem, in den biologischen Körper führenden Insufflationsinstrument (19) die vorgesehene Temperatur erreicht.

2. Erwärmungsvorrichtung nach Anspruch 1,
dadurch **gekennzeichnet,**
daß die Heizeinrichtung (11) einen integrierten Heizdraht, insbesondere als Heizdrahtwicklung, aufweist, die insbesondere in der Nähe einer Innenwandungsfläche (15) der Schlauchleitung (10) vorgesehen ist.

3. Erwärmungsvorrichtung nach Anspruch 1 oder 2,
dadurch **gekennzeichnet,**
daß die Schlauchleitung (10) mindestens im Innenbereich eine medizinisch geeignete Silikonschicht (13) aufweist, in oder um die die Heizdrahtwicklung vorgesehen ist.

4. Erwärmungsvorrichtung nach Anspruch 2 oder 3,
dadurch **gekennzeichnet,**
daß die Schlauchleitung (10) eine insbesondere extrudierte innere und äußere Schlauchlage (13, 14) aus Kunststoff, insbesondere aus einem Silikonmaterial oder PU-Material, aufweist, zwischen denen mindestens die Heizdrahtwicklung vorgesehen ist.

5. Erwärmungsvorrichtung nach einem der Ansprüche 2 bis 4,
dadurch **gekennzeichnet,**
daß die Heizdrahtwicklung am distalen Bereich (17) der Schlauchleitung (10) abgesetzt ist, und
daß in diesem Bereich (17) ein Temperaturfühler (21) vorgesehen ist, dessen Fühlerleitung in die Schlauchleitung (10) integriert, insbesondere mitextrudiert, ist.

6. Erwärmungsvorrichtung nach einem der Ansprüche 1 bis 5,
dadurch **gekennzeichnet,**
daß die Schlauchleitung (10) eine Länge von 1,8 bis 2,5 m entsprechend den klinischen Bedingungen, aufweist.

7. Erwärmungsvorrichtung nach einem der Ansprüche 1 bis 6,
dadurch **gekennzeichnet,**
daß der Heizeinrichtung (11) eine Heizleistung von etwa 20 W zur Erwärmung des Gases von 20°C auf 30°C bis 37°C zugeführt ist.

8. Erwärmungsvorrichtung nach einem der Ansprüche 1 bis 7,
dadurch **gekennzeichnet,**
daß distalseitig an der Schlauchleitung (10) ein Torsionskupplungsglied (18), insbesondere vor einer nachfolgenden Veress-Nadel (19), vorgesehen ist.

9. Erwärmungsvorrichtung nach einem der Ansprüche 1 bis 8,
dadurch **gekennzeichnet,**
daß eine direkte elektrische Heizleitungsverbindung zwischen der Heizeinrichtung (11) und dem Insufflationsgerät (8) besteht oder die Heizeinrichtung (11) über eine Trafoeinheit (6) leistungsbeaufschlagt ist.

10. Erwärmungsvorrichtung nach einem der Ansprüche 1 bis 9,
dadurch **gekennzeichnet,**
daß die Heizeinrichtung (11) als Kaltleiter mit konstanter Stromaufnahme nach einer Aufheizzeit von etwa 10 min ausgelegt ist.

## Claims

1. A heating apparatus for a gas to be introduced in a biological body, in particular CO₂-gas, for an insufflator (8) with a heating device (11), with which the gas can be heated to a predetermined temperature, and with a hose line (10) leading from the insufflator (8) to the biological body,
**characterized** in that
the heating device (11) is incorporated in the hose line (10), and
the heating device (11) is controllable so that the gas reaches the predetermined temperature directly before an insufflator (19) leading into the biological body.

2. A heating apparatus according to claim 1,
**characterized** in that
the heating device (11) has an integrated heating wire, in particular as a heating wire coil, which is provided in particular in the vicinity of the inner wall surface (15) of the hose line (10).

3. A heating apparatus according to claim 1 or 2,
**characterized** in that
the heating hose (10) has at least in the inner area a medically suitable silicon layer (13) in or around which a heating wire coil is provided.

4. A heating apparatus according to claim 2 or 3,
**characterized** in that
the hose line (10) has an, in particular extruded, inner and outer plastic hose layer (13, 14), in particular made from a silicon material or a PU material, between which at least the heating wire coil is provided.

5. A heating apparatus according to claims 2 to 4,
**characterized** in that
the heating wire coil is offset in the distal area (17) of the hose line (10), and
that in this area (17) a temperature sensor (21) is provided, whose sensor line is integrated into the hose line (10), in particular extruded in the hose line.

6. A heating apparatus according to any of the claims 1 to 5,
**characterized** in that
the hose line (10) has a length of 1.8 m to 2.5 m corresponding to the clinical conditions.

7. A heating apparatus according to any of the claims 1 to 6,
**characterized** in that
the heating device (11) is supplied a heating power of approximately 20 W for heating the gas from 20° C to 30° to 37° C.

8. A heating device according to any of the claims 1 to 7,
**characterized** in that
on the distal side of the hose line (10) a torsional coupling (18) is provided, in particular before a following Veress needle (19).

9. A heating apparatus according to any of the claims 1 to 8,
**characterized** in that
a direct electrical heating line connection is provided between the heating device (11) and the insufflator (8) or power is supplied by a transformer unit (6) to the heating device (11).

10. A heating apparatus according to any of the claims 1 to 9,
**characterized** in that
the heating device (11) is constructed as a PTC resistor with a constant power consumption following a heating up time of approximately 10 min.

## Revendications

1. Dispositif de chauffage pour un gaz, notamment du CO₂, qui est introduit dans un corps biologique pour un appareil d'insufflation (8) comprenant une installation de chauffage (11) à l'aide de laquelle le gaz peut être chauffé à une température prédéterminée et un tuyau (10) reliant l'appareil d'insufflation (8) au corps biologique, caractérisé en ce que
- l'installation de chauffage (11) est réalisée dans le tuyau (10) et
- l'installation de chauffage (11) est commandée pour que le gaz soit à la température prévue directement avant l'instrument d'insufflation (19) conduisant dans le corps biologique.

2. Dispositif de chauffage selon la revendication 1, caractérisé en ce que l'installation de chauffage (11) a un fil chauffant intégré, notamment un enroulement à fil chauffant prévu notamment au voisinage de la surface de la paroi intérieure (15) du tuyau (10).

3. Dispositif de chauffage selon la revendication 1 ou 2, caractérisé en ce que le tuyau (10) est revêtu au moins intérieurement d'une couche de silicium medicalement propre (13) et est prévu dans ou autour de l'enroulement de fil chauffant.

4. Dispositif de chauffage selon la revendication 2 ou 3, caractérisé en ce que le tuyau (10) comporte une couche intérieure et extérieure (13, 14) notamment extrudées en matière plastique, notamment en silicone ou en polyuréthane, entre lesquelles il y a au moins un enroulement de fil chauffant.

5. Dispositif de chauffage selon l'une des revendications 2 à 4, caractérisé en ce que l'enroulement de fil chauffant est dégagé de la zone éloignée (17) du tuyau (10) et dans cette zone (17) il est prévu un détecteur de température (21) dont la ligne de détection est intégrée au tuyau (10), notamment par extrusion.

6. Dispositif de chauffage selon l'une des revendications 1 à 5, caractérisé en ce que le tuyau (10) a une longueur 1,8 à 2,5 m selon les conditions cliniques.

7. Dispositif de chauffage selon l'une des revendications 1 à 6, caractérisé en ce qu'à l'installation de chauffage (11) une installation de chauffage d'une puissance d'environ 20 W est pour chauffer le gaz de 20° C à 30°C - 37°C est amenée.

8. Dispositif de chauffage selon l'une des revendications 1 à 7, caractérisé en ce que du côté éloigné du tuyau (10) il est prévu un organe d'accouplement à torsion (18), notamment en amont d'une aiguille Veress (19) suivante.

9. Dispositif de chauffage selon l'une des revendications 1 à 8, caractérisé par l'existence d'une liaison de chauffage électrique directe entre l'installation de chauffage (11) et l'appareil d'insufflation (8) ou l'installation (11) est alimentée par l'intermédiaire d'un ensemble à transformateur (6).

10. Dispositif de chauffage selon l'une des revendications 1 à 9, caractérisé en ce que l'installation de chauffage (11) est un conducteur froid absorbant une intensité électrique constante après une durée de montée en température de l'ordre de 10 minutes.
